# EUROPEAN PATENT APPLICATION

(11) **EP 4 428 135 A1**
(43) Date of publication of application: **11.09.2024**
(21) Application number: 24169552.7
(22) Date of filing: 17.05.2023
(51) Int. Cl.: C07D 487/04, A61P 31/14, A61K 31/53

(54) **DEUTERATED NUCLEOSIDE COMPOUNDS AND USE THEREOF**

(30) Priority: 17.05.2022 CN 202210548373; 15.06.2022 CN 202210695557; 19.08.2022 CN 202211002496; 07.03.2023 CN 202310213459
(62) Divisional of application: 23807006.4
(71) Applicant: Medshine Discovery Inc., Nanjing, Jiangsu 210032 (CN)
(72) Inventor: LI, Peng, Shanghai, 200131 (CN); LI, Xiaolin, Shanghai, 200131 (CN); YANG, Yaxun, Shanghai, 200131 (CN); LUO, Zhi, Shanghai, 200131 (CN); HE, Haiying, Shanghai, 200131 (CN); CHEN, Shuhui, Shanghai, 200131 (CN)
(74) Representative: Dehns

(57) **Abstract**

Disclosed are a series of deuterated nucleoside compounds and a use thereof. Specifically disclosed are compounds represented by formula (VI-1), stereoisomers or pharmaceutically acceptable salts thereof.

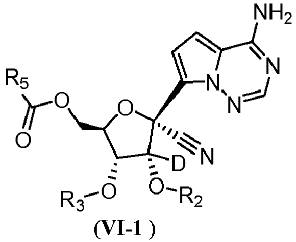

## Description

The present application is a Divisional Application of EP Application No. 23807006.4, filed on February 5, 2024, which claims priorities of the Chinese Patent Application No. CN202210548373.7, filed on May 17, 2022, the Chinese Patent Application No. CN202210695557.6, filed on June 15, 2022, the Chinese Patent Application No. CN202211002496.7, filed on August 19, 2022 and the Chinese Patent Application No. CN202310213459.9, filed on March 7, 2023, the contents of which are incorporated herein by reference in their entireties.

### TECHNICAL FIELD

The present disclosure relates to the technical field of medicinal chemistry, especially relates to a series of deuterated nucleoside compounds and a use thereof. The present disclosure specifically relates to a compound of formula (VI-1), a stereoisomer thereof, or a pharmaceutically acceptable salt thereof.

### BACKGROUND

Currently, there are seven known coronaviruses that can infect humans, namely HCoV-229E, HCoV-OC43, HCoV-NL63, HCoV-HKU1, SARS-CoV (SARS), MERS-CoV (MERS), and SARS-CoV-2 (COVID-2019) which emerged at the end of 2019. The latter three may cause more severe symptoms and even lead to death in infected individuals. Coronavirus infections, particularly COVID-2019 and its variants, spread rapidly, and some patients may require an extended recovery period, significantly impacting their work and daily lives. There is an urgent need for more practical and effective drugs and methods to treat such infectious diseases.

### CONTENT OF THE PRESENT INVENTION

In one aspect, the present disclosure provides a compound of formula (VI-1), a stereoisomer thereof, or a pharmaceutically acceptable salt thereof, 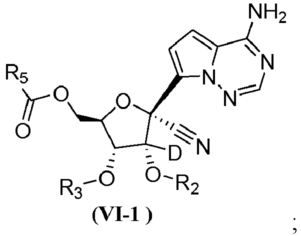 wherein
R₂ and R₃ are each independently selected from hydrogen, C₁₋₆ alkyl-C(=O)-, C₁₋₄ alkoxy-C(=O)-, and phenyl-C(=O)-, and the C₁₋₆ alkyl, C₁₋₄ alkoxy, and phenyl are each independently and optionally substituted by 1, 2, or 3 R;
R₅ is selected from phenyl, and the phenyl is optionally substituted by 1, 2, or 3 R;
each R is independently selected from hydroxyl, halogen, amino, and cyano.

In some embodiments of the present disclosure, each R is independently selected from hydroxyl, and other variables are as defined in the present disclosure.

In some embodiments of the present disclosure, in the compound of formula (VI-1), the stereoisomer thereof, or the pharmaceutically acceptable salt thereof, R₅ is selected from 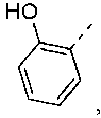 and other variables are as defined in the present disclosure.

In some embodiments of the present disclosure, the R₂ and R₃ are each independently selected from hydrogen, isopropyl-C(=O)-, and phenyl-C(=O)-, and the isopropyl and phenyl are each independently and optionally substituted by 1, 2, or 3 R, and other variables are as defined in the present disclosure.

In some embodiments of the present disclosure, the R₂ and R₃ are each independently selected from hydrogen, isopropyl-C(=O)-, and 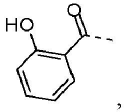 and other variables are as defined in the present disclosure.

In some embodiments of the present disclosure, the R₂ and R₃ are each independently selected from hydrogen and isopropyl-C(=O)-, and other variables are as defined in the present disclosure.

In some embodiments of the present disclosure, the R₂ and R₃ are each independently selected from hydrogen and 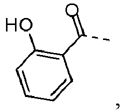 and other variables are as defined in the present disclosure.

There are still some embodiments of the present disclosure which are obtained by any combination of the above variables.

The present disclosure also provides a compound of the following formula or a pharmaceutically acceptable salt thereof, selected from:

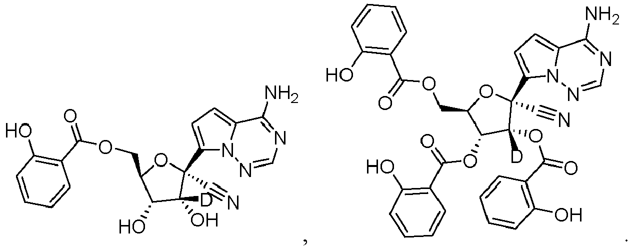

In some embodiments of the present disclosure, a use of the compound or the pharmaceutically acceptable salt thereof in the manufacture of a medicament for treating diseases related to RNA-dependent RNA polymerase inhibitor is provided.

In some embodiments of the present disclosure, the use is characterized in that the disease related to RNA-dependent RNA polymerase inhibitor is a viral infectious disease, such as viral influenza, viral pneumonia.

In some embodiments of the present disclosure, the use is characterized in that the disease related to RNA-dependent RNA polymerase inhibitor is viral pneumonia.

### Technical effect

The compound of the present disclosure exhibits potent anti-coronavirus activity *in vitro*; upon administration to mice, the compound of the present disclosure is rapidly metabolized *in vivo* into its corresponding active metabolites, exerting a pharmacological effect; the pharmaceutical concentration of the compound of the present disclosure is sustained for a longer duration and maintained at a higher concentration, which is conducive to the continuous inhibition of the virus and the exertion of pharmacological efficacy.

### Definition and description

Unless otherwise specified, the following terms and phrases when used herein have the following meanings. A specific term or phrase should not be considered indefinite or unclear in the absence of a particular definition, but should be understood in the ordinary sense. When a trading name appears herein, it is intended to refer to its corresponding commodity or active ingredient thereof.

The term "pharmaceutically acceptable" is used herein in terms of those compounds, materials, compositions, and/or dosage forms, which are suitable for use in contact with human and animal tissues within the scope of reliable medical judgment, with no excessive toxicity, irritation, an allergic reaction, or other problems or complications, commensurate with a reasonable benefit/risk ratio.

Unless otherwise specified, the term "treatment" is intended to refer to all processes that may slow down, interrupt, contain, or prevent the progression of a disease, but does not necessarily imply the complete elimination of all symptoms.

The term "pharmaceutically acceptable salt" refers to a salt of the compound of the present disclosure that is prepared by reacting the compound having a specific substituent of the present disclosure with a relatively non-toxic acid or base. When the compound of the present disclosure contains a relatively acidic functional group, a base addition salt can be obtained by bringing the neutral form of the compound into contact with a sufficient amount of base in a pure solution or a suitable inert solvent. The pharmaceutically acceptable base addition salt includes a salt of sodium, potassium, calcium, ammonium, organic amine, magnesium, or similar salts. When the compound of the present disclosure contains a relatively basic functional group, an acid addition salt can be obtained by bringing the neutral form of the compound into contact with a sufficient amount of acid in a pure solution or a suitable inert solvent. Certain specific compounds of the present disclosure contain both basic and acidic functional groups, thus can be converted to any base or acid addition salt.

The pharmaceutically acceptable salt of the present disclosure can be prepared from the parent compound that contains an acidic or basic moiety by conventional chemical method. Generally, such salt can be prepared by reacting the free acid or base form of the compound with a stoichiometric amount of an appropriate base or acid in water or an organic solvent or a mixture thereof.

Unless otherwise specified, the term "isomer" is intended to include a geometric isomer, a cis-trans isomer, a stereoisomer, an enantiomer, an optical isomer, a diastereoisomer, and a tautomericisomer.

The compounds of the present disclosure may exist in specific geometric or stereoisomeric forms. The present disclosure contemplates all such compounds, including cis and trans isomers, (-)- and (+)-enantiomers, (*R*)- and (*S*)-enantiomers, diastereoisomers, (D)-isomers, (Z)-isomers, racemic, and other mixtures thereof, such as enantiomers or diastereomer enriched mixtures, all of which are within the scope of the present disclosure. Additional asymmetric carbon atoms may be present in substituents such as alkyl. All these isomers and their mixtures are included within the scope of the present disclosure.

Unless otherwise specified, the term "enantiomer" or "optical isomer" refers to stereoisomers that are mirror images of each other.

Unless otherwise specified, the term *"cis-trans* isomer" or "geometric isomer" is caused by the inability to rotate freely of double bonds or single bonds of ring-forming carbon atoms.

Unless otherwise specified, the term "diastereomer" refers to a stereoisomer in which a molecule has two or more chiral centers and the relationship between the molecules is not mirror images.

Unless otherwise specified, "(+)" refers to dextrorotation, "(-)" refers to levorotation, and or "(±)" refers to racemic.

Unless otherwise specified, the absolute configuration of a stereogenic center is represented by a wedged solid bond ( 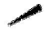 ) and a wedged dashed bond ( 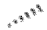 ), and the relative configuration of a stereogenic center is represented by a straight solid bond ( 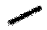 ) and a straight dashed bond ( 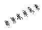 ), a wave line ( 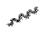 ) is used to represent a wedged solid bond ( 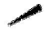 ) or a wedged dashed bond ( 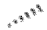 ), or the wave line ( 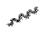 ) is used to represent a straight solid bond ( 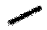 ) or a straight dashed bond ( 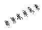 ).

Unless otherwise specified, when a group has one or more linkable sites, any one or more sites of the group can be linked to other groups through chemical bonds. When the linking site of the chemical bond is not positioned, and there is an H atom at the linkable site, then the number of H atoms at the site will decrease correspondingly with the number of the chemical bonds linking thereto so as to meet the corresponding valence. The chemical bond between the site and other groups can be represented by a straight solid bond ( 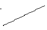 ), a straight dashed bond 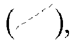 or a wavy line 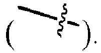 For example, the straight solid bond in -OCH₃ means that it is linked to other groups through the oxygen atom in the group; the straight dashed bond in 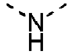 means that it is linked to other groups through the two ends of nitrogen atom in the group; the dashed bond in 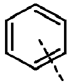 means that the phenyl group can be linked to other groups through one chemical bond at any connectable site in the phenyl group.

The compounds of the present disclosure may exist in specific forms. Unless otherwise specified, the term "tautomer" or "tautomeric form" means that at room temperature, the isomers of different functional groups are in dynamic equilibrium and can be transformed into each other quickly. If tautomers possibly exist (such as in solution), the chemical equilibrium of tautomers can be reached. For example, proton tautomer (also called prototropic tautomer) includes interconversion through proton migration, such as keto-enol isomerization and imine-enamine isomerization. Valence tautomer includes some recombination of bonding electrons for mutual transformation. A specific example of keto-enol tautomerization is the tautomerism between two tautomers of pentane-2,4-dione and 4-hydroxypent-3-en-2-one.

Unless otherwise specified, the terms "enriched in one isomer", "enriched in isomers", "enriched in one enantiomer" or "enriched in enantiomers" refer to the content of one of the isomers or enantiomers is less than 100%, and the content of the isomer or enantiomer is greater than or equal to 60%, or greater than or equal to 70%, or greater than or equal to 80%, or greater than or equal to 90%, or greater than or equal to 95%, or greater than or equal to 96%, or greater than or equal to 97%, or greater than or equal to 98%, or greater than or equal to 99%, or greater than or equal to 99.5%, or greater than or equal to 99.6%, or greater than or equal to 99.7%, or greater than or equal to 99.8%, or greater than or equal to 99.9%.

Unless otherwise specified, the term "isomer excess" or "enantiomeric excess" refers to the difference between the relative percentages of two isomers or two enantiomers. For example, if the content of one isomer or enantiomer is 90%, and the content of the other isomer or enantiomer is 10%, the isomer or enantiomer excess (ee value) is 80%.

Optically active (*R*)- and (*S*)-isomers, or *D* and *L* isomers can be prepared using chiral synthesis, chiral reagents, or other conventional techniques. If one kind of enantiomer of certain compound of the present disclosure is to be obtained, it can be obtained by asymmetric synthesis or derivative action of chiral auxiliary, wherein the resulting diastereomeric mixture is separated and the auxiliary group is cleaved to provide the pure desired enantiomer. Alternatively, when the molecule contains a basic functional group (such as amino) or an acidic functional group (such as carboxyl), a salt of a diastereoisomer is formed with an appropriate optically active acid or base, and then diastereomeric resolution is performed by conventional methods known in the art, and then the pure enantiomer is recovered. In addition, the enantiomer and the diastereoisomer are generally separated through chromatography which uses a chiral stationary phase and optionally combines with a chemical derivative method (such as carbamate generated from amine).

The compound of the present disclosure may contain an unnatural proportion of atomic isotope at one or more atoms that constitute the compound. For example, the compound can be radiolabeled with a radioactive isotope, such as tritium (³H), iodine-125 (¹²⁵I), or C-14 (¹⁴C). For another example, deuterated drugs can be formed by replacing hydrogen with deuterium, the bond formed by deuterium and carbon is stronger than that of ordinary hydrogen and carbon, compared with non-deuterated drugs, deuterated drugs have the advantages of reduced toxic and side effects, increased drug stability, enhanced efficacy, extended biological half-life of drugs, etc. All isotopic variations of the compound of the present disclosure, whether radioactive or not, are encompassed within the scope of the present disclosure.

"Optional" or "optionally" means that the subsequent event or condition may occur but not requisite, that the term includes the instance in which the event or condition occurs and the instance in which the event or condition does not occur.

The term "substituted" means one or more than one hydrogen atom on a specific atom is substituted by the substituent, including deuterium and hydrogen variables, as long as the valence of the specific atom is normal and the substituted compound is stable. When the substituent is an oxygen (i.e., =O), it means two hydrogen atoms are substituted. Positions on an aromatic ring cannot be substituted with a ketone. The term "optionally substituted" means an atom can be substituted with a substituent or not, unless otherwise specified, the type and number of the substituent may be arbitrary as long as being chemically achievable.

When any variable (such as R) occurs in the constitution or structure of the compound more than once, the definition of the variable at each occurrence is independent. Thus, for example, if a group is substituted by 0 to -2 R, the group can be optionally substituted with up to two R, wherein the definition of R at each occurrence is independent. Moreover, a combination of the substituent and/or the variant thereof is allowed only when the combination results in a stable compound.

Unless otherwise specified, the term "C₁₋₆ alkyl" refers to a linear or branched saturated hydrocarbon group consisting of 1 to 6 carbon atoms. The C₁₋₆ alkyl includes C₁₋₅, C₁₋₋₄, C₁₋₋₃, C₁₋₂, C₂₋₆, C₂₋₋₄, C₆, C₅ alkyl, etc.; it can be monovalent (such as methyl), divalent (such as methylene), or multivalent (such as methine). Examples of C₁₋₆ alkyl include, but are not limited to, methyl (Me), ethyl (Et), propyl (including *n*-propyl and isopropyl), butyl (including *n*-butyl, isobutyl, *s*-butyl, and *t*-butyl), pentyl (including *n*-pentyl, isopentyl, and neopentyl), hexyl, etc.

Unless otherwise specified, the term "C₁₋₄ alkoxy" refers to an alkyl group containing 1 to 4 carbon atoms that are connected to the rest of the molecule through an oxygen atom. The C₁₋₄ alkoxy includes C₁₋₃, C₁₋₂, C₂₋₄, C₄, C₃ alkoxy, etc. Examples of C₁₋₆ alkoxy include, but are not limited to, methoxy, ethoxy, propoxy (including *n*-propoxy and isopropoxy), butoxy (including *n*-butoxy, isobutoxy, *s*-butoxy, and *t*-butoxy), pentyloxy (including *n*-pentyloxy, isopentyloxy, and neopentyloxy), hexyloxy, etc.

Unless otherwise specified, the term "halogen element" or "halogen" by itself or as part of another substituent refers to fluorine, chlorine, bromine, or iodine atom.

The compounds of the present disclosure can be prepared by a variety of synthetic methods known to those skilled in the art, including the specific embodiments listed below, the embodiments formed by their combination with other chemical synthesis methods, and equivalent alternatives known to those skilled in the art, preferred embodiments include but are not limited to the examples of the present disclosure.

The structure of the compounds of the present disclosure can be confirmed by conventional methods known to those skilled in the art, and if the present disclosure involves an absolute configuration of a compound, then the absolute configuration can be confirmed by means of conventional techniques in the art. For example, in the case of single crystal X-ray diffraction (SXRD), diffraction intensity data are collected from the cultured single crystal using a Bruker D8 venture diffractometer with CuKα radiation as the light source and scanning mode: ϕ/ω scan, and after collecting the relevant data, the crystal structure is further analyzed by direct method (Shelxs97), so that the absolute configuration can be confirmed.

The solvent used in the present disclosure is commercially available. The present disclosure uses the following abbreviations: aq stands for water; eq stands for equivalent; M stands for mol/L; DCM stands for dichloromethane; PE stands for petroleum ether; DMF stands for N,N-dimethylformamide; DMSO stands for dimethyl sulfoxide; EtOAc stands for ethyl acetate; EtOH stands for ethanol; MeOH stands for methanol; THF stands for tetrahydrofuran; HCOOH stands for formic acid; TFA stands for trifluoroacetic acid; Py stands for pyridine; DIPEA stands for diisopropylethylamine; DMP stands for Dess-Martin periodinane; NaBD₄ stands for sodium borodeuteride; MeOD stands for deuterated methanol; TBAF stands for tetrabutylammonium fluoride; ACN stands for acetonitrile; mp stands for melting point; RdRp stands for RNA-dependent RNA polymerase; room temperature stands for 25°C.

The compounds of the present disclosure are named according to the conventional naming principles in the art or by ChemDraw^{®} software, and the commercially available compounds use the supplier catalog names.

### DETAILED DESCRIPTION OF THE PREFERRED EMBODIMENT

The present disclosure is described in detail by the examples below, but it does not mean that there are any adverse restrictions on the present disclosure. The present disclosure has been described in detail herein, and its specific examples have also been disclosed; for one skilled in the art, it is obvious to make various modifications and improvements to the examples of the present disclosure without departing from the spirit and scope of the present disclosure.

### Example 1

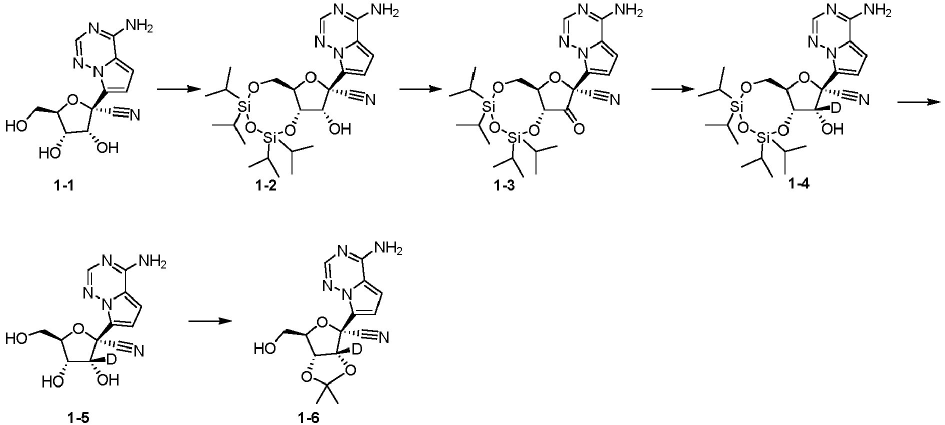

### Step 1: Synthesis of compound 1-2

To a reaction flask were added compound **1-1** (3.0 g) and dichloromethane (20 mL), then sequentially added pyridine (29.40 g) and compound 1,3-dichloro-1,1,3,3-tetraisopropyldisiloxane (3.90 g). The reaction system was stirred at room temperature for 14 hours. The reaction mixture was directly concentrated under reduced pressure to obtain a crude product. The crude product was separated by flash column chromatography (ISCO^{®}; 40 g SepaFlash^{®} Silica Flash Column, mobile phase: 0 to 50% EtOAc/PE, flow rate: 50 mL/min). Thus compound 1-2 (purity: 84.0%) was obtained.

MS m/z(ESI): [M+H]⁺ =534.6.

### Step 2: Synthesis of compound 1-3

To a reaction flask were added compound **1-2** (1.0 g, purity: 84%) and dichloromethane (50 mL), then added Dess-Martin periodinane (1.19 g). The reaction system was stirred at room temperature for 4 hours. The reaction mixture was filtered and directly concentrated under reduced pressure to obtain a crude product. The crude product was separated by flash column chromatography (ISCO^{®}; 12 g SepaFlash^{®} Silica Flash Column, mobile phase: 0 to 33% EtOAc/PE, flow rate: 35 mL/min). Thus compound **1-3** was obtained.

MS m/z(ESI): [M+H]⁺ =532.2.

### Step 3: Synthesis of compound 1-4

To a reaction flask were added compound **1-3** (200 mg) and deuterated methanol (10 mL), then added sodium borodeuteride (28.46 mg). The reaction system was stirred at room temperature for 1 hour. The reaction mixture was directly concentrated under reduced pressure to obtain a crude product. The crude product was separated by flash column chromatography (ISCO^{®}; 12 g SepaFlash^{®} Silica Flash Column, mobile phase: 0 to 30% EtOAc/PE, flow rate: 30 mL/min). Thus compound **1-4** was obtained.

MS m/z(ESI): [M+H]⁺ =535.2.

### Step 4: Synthesis of compound 1-5

To a reaction flask were added compound **1-4** (110 mg) and tetrahydrofuran (4 mL), then added a solution of tetrabutylammonium fluoride in tetrahydrofuran (1 M, 411.39 µL). The reaction system was stirred at room temperature for 0.5 hours. The reaction mixture was directly concentrated under reduced pressure to obtain a crude product. Thus compound **1-5** was obtained.

MS m/z(ESI): [M+H]⁺ =293.2.

¹H NMR (400MHz, DMSO-d6) δ =7.88 (s, 1H), 6.91-6.94 (m, 2H) ,3.95 (d, J=5.3 Hz, 1H), 3.65 (br d, J=3.5 Hz, 1H), 3.62 (br d, J=3.3 Hz, 2H), 3.52 (br d, J=4.5 Hz, 2H).

### Step 5: Synthesis of compound 1-6

To a reaction flask were added compound **1-5** (100 mg) and acetone (40 mL), then sequentially added sulfuric acid (50.34 mg) and compound 2,2-dimethoxypropane (178.17 mg). The reaction system was stirred at 45°C for 0.5 hours. Water (50 mL) was added to the reaction system, and the reaction mixture was adjusted to neutral using a 15% sodium bicarbonate solution. The mixture was then extracted with EtOAc (50 mL * 3). The organic phases were combined and subsequently washed sequentially with saturated brine (50 mL) and water (50 mL). After the phases were separated, the organic phase was dried over anhydrous Na₂SO₄, and finally, the organic phase was directly concentrated under reduced pressure. Thus compound **1-6** was obtained.

MS m/z(ESI): [M+H]⁺ =333.2.

### Example 2

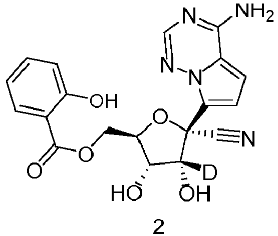

### Synthetic route:

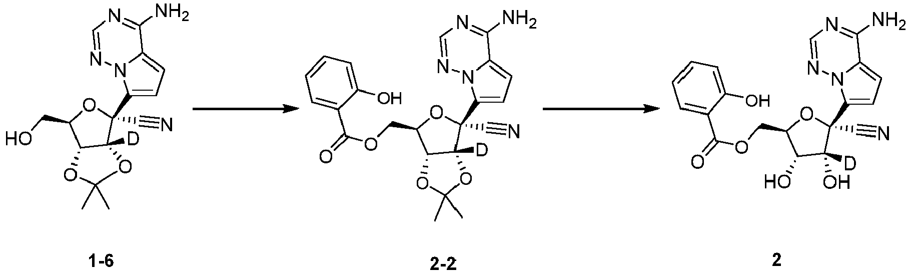

### Step 1: Synthesis of compound 2-2

To a reaction flask were added compound 1-6 (300 mg), salicylic acid (311.71 mg), and dichloromethane (3 mL), then sequentially added 4-dimethylaminopyridine (275.71 mg) and *N,N-*dicyclohexylcarbodiimide (465.63 mg). The reaction system was stirred at 35°C for 12 hours. The reaction system was quenched by water (30 mL), and extracted with EtOAc (10 mL * 2). The origanic phases were combined, washed with saturated brine (10 mL), and dried over anhydrous Na₂SO₄. The organic phase was filtered, and concentrated under reduced pressure. The crude product was separated by flash column chromatography (ISCO^{®}; 4 g SepaFlash^{®} Silica Flash Column, mobile phase: 0 to 80% ethyl acetate/petroleum ether, flow rate: 18 mL/min) to obtain compound 2-2.

MS m/z(ESI): [M+H] ⁺ =453.0.

### Step 2: Synthesis of compound 2

To a reaction flask were added compound 2-2 (275 mg) and water (3 mL), then added formic acid (15 mL). The reaction system was stirred at room temperature for 4 hours. The reaction mixture was directly concentrated under reduced pressure to obtain a crude product. The crude product was purified by prep-HPLC (column: O-Welch C18 150 * 30 mm * 5 µm; mobile phase: [H₂O(FA)-ACN]; ACN%: 18% to 58%, 10 min) to obtain compound 2.

MS m/z(ESI): [M+H]⁺ =413.3.

¹H NMR: (400MHz, DMSO-d₆) δ = 10.43 (s, 1H), 7.98 - 7.81 (m, 2H), 7.64 (d, *J*=6.8 Hz, 1H), 7.53 (t, *J*=7.7 Hz, 1H), 6.98 (d, *J*=8.3 Hz, 1H), 6.91 (t, *J*=7.7 Hz, 1H), 6.87 (d, *J*=4.5 Hz, 1H), 6.80 (d, *J*=4.5 Hz, 1H), 6.34 (s, 1H), 5.46 (br s, 1H), 4.63 (dd, *J*=2.6, 12.2 Hz, 1H), 4.48 - 4.43 (m, 1H), 4.41 - 4.36 (m, 1H), 4.13 (br d, *J*=6.3 Hz, 1H).

### Bioassay:

### Test example 1: Pharmacokinetic evaluation of compounds

Experimental objective: To test the pharmacokinetics of the compounds in CD-1 mice

Experimental materials: CD-1 mice (male, from Beijing Vital River Laboratory Animal Technology Co., Ltd.)

Experimental procedure: The pharmacokinetic characteristics in rodents after intragastric administration of the compound were tested using a standard protocol. In the experiment, candidate compounds are prepared into clear solutions (the solvent for intragastric administration preparations was 1% methylcellulose 4000 aqueous solution). Four male CD-1 mice were used in this project, with a drug concentration of 10 mg/mL and a dose of 100 mg/kg. Plasma samples were collected at 0 h (before administration) and post-administration at 0.083, 0.25, 0.5, 1, 2, 4, 8, 24 h. Samples were centrifuged at 3200 g for 10 minutes at 4°C, and the supernatant was separated to obtain plasma samples. The plasma was transferred to a pre-cooled centrifuge tube, snap-frozen in dry ice, and then stored in an ultra-low temperature freezer at -70 ± 10°C/-60°C or lower. Plasma drug concentration was quantitatively analyzed by LC-MS/MS analysis method, and the plasma drug concentration data of the metabolite of the compound of the present disclosure were processed by using the pharmacokinetic software of WinNonlin Version 6.3 or above (Pharsight) in a non-compartmental model. Relevant pharmacokinetic parameters such as peak concentration (Cₘₐₓ), half-life (T_{1/2}), and area under the curve (AUC_{0-inf}) were calculated using the linear-log trapezoidal method. Experimental results are shwn in Tables 1 and 2.

**Table 1: Pharmacokinetic experimental data**

| Pharmacokinetic Parameter | Compound 2 |
|---|---|
| Cₘₐₓ (nmol/L) | 38150 |
| T_{1/2} (h) | 2.67 |
| AUC_{0-inf} (h * nmol/L) | 150291 |

| | |
|---|---|
| Note: The compounds of the present disclosure would be rapidly metabolized *in vivo,* and the above data are all for the detected metabolites. | |

**Table 2: Concentration of nucleoside Nuc in plasma and tissues at different times**

| Nucleoside PK Assay | Concentration of Nucleoside Nuc in Plasma and Tissues (nM) |
|---|---|
| Time (h) | Compound 2 |
| 1 | 38150 |
| 2 | 32700 |
| 4 | 21500 |
| 8 | 1489 |
| 24 | 95.8 |

**Conclusion:** After administration of the compound of the present disclosure to mice, it is rapidly metabolized *in vivo* into its corresponding active metabolites, exerting a pharmacological effect; the drug concentration of the compound of the present disclosure is sustained for a long duration and maintained at a high concentration, which is conducive to the continuous inhibition of the virus and the exertion of pharmacological efficacy.

## Claims

1. A compound of formula (VI-1), a stereoisomer thereof, or a pharmaceutically acceptable salt thereof, 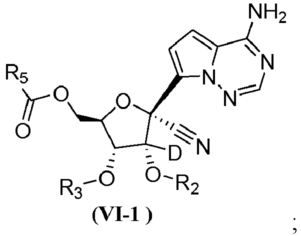 wherein
R₂ and R₃ are each independently selected from hydrogen, C₁₋₆ alkyl-C(=O)-, C₁₋₄ alkoxy-C(=O)-, and phenyl-C(=O)-, and the C₁₋₆ alkyl, C₁₋₄ alkoxy, and phenyl are each independently and optionally substituted by 1, 2, or 3 R;
R₅ is selected from phenyl, and the phenyl is optionally substituted by 1, 2, or 3 R;
each R is independently selected from hydroxyl, halogen, amino, and cyano.

2. The compound, the stereoisomer thereof, or the pharmaceutically acceptable salt thereof according to claim 1, wherein each R is independently selected from hydroxyl.

3. The compound, the stereoisomer thereof, or the pharmaceutically acceptable salt thereof according to claim 1, wherein R₅ is selected from

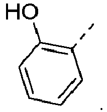

4. The compound, the stereoisomer thereof, or the pharmaceutically acceptable salt thereof according to claim 1, wherein R₂ and R₃ are each independently selected from hydrogen, isopropyl-C(=O)-, and phenyl-C(=O)-, and the isopropyl and phenyl are each independently and optionally substituted by 1, 2, or 3 R.

5. The compound, the stereoisomer thereof, or the pharmaceutically acceptable salt thereof according to claim 4, wherein R₂ and R₃ are each independently selected from hydrogen, isopropyl-C(=O)-, and

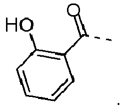

6. The compound, the stereoisomer thereof, or the pharmaceutically acceptable salt thereof according to claim 1, wherein the compound is

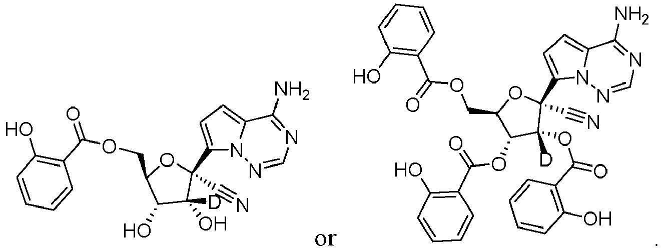

7. The compound, the stereoisomer thereof or the pharmaceutically acceptable salt thereof according to any one of claims 1-6 for use in treating a viral infectious disease.

8. The compound, the stereoisomer thereof or the pharmaceutically acceptable salt thereof for use according to claim 7, wherein the viral infectious disease is viral influenza or viral pneumonia.
